# EUROPEAN PATENT APPLICATION

(11) **EP 4 647 094 A1**
(43) Date of publication of application: **12.11.2025**
(21) Application number: 24305715.5
(22) Date of filing: 07.05.2024
(51) Int. Cl.: A61M 5/315

(54) **PLUNGER ASSEMBLY WITH STROKE LIMITER FOR A SYRINGE ASSEMBLY**

(71) Applicant: Becton Dickinson France, 38800 Le Pont-de-Claix (FR)
(72) Inventor: GAGLIANO, Julien, 38240 MEYLAN (FR); LE DIMET, Gwenn, 38850 Charavines (FR)
(74) Representative: Germain Maureau

(57) **Abstract**

The invention relates to a plunger assembly (104) for a syringe assembly (100) comprising a syringe body (102), wherein the plunger assembly (104) comprises:
- a plunger rod (102) extending along a longitudinal axis (A) and having a proximal end (126) and a distal end (123);
- at the distal end of the plunger rod, a stopper mount portion (123) for receiving a stopper (124);
characterized in that the plunger assembly (104) comprises a plunger stroke limiter (128, 158), in that the position of the plunger stroke limiter (128, 158) with respect to the stopper mount portion (123) is user-adjustable along the longitudinal axis, and in that the plunger stroke limiter (123) is configured to abut against a syringe body abutment surface (130) of the syringe body (102) for at least one position of the plunger stroke limiter (128, 158) with respect to the stopper mount portion (123).

## Description

### Technical Domain

The invention relates to the field of syringes, such as prefilled or pre-fillable syringes, especially syringes for delivering a drug to a patient.

### Prior art

A syringe comprises a hollow tubular body, hereinafter called barrel, which has a main tubular portion forming a container for a medical product, typically a liquid drug. The barrel has, along the longitudinal axis, a proximal end, a distal end, and a distal tip at its distal end. The barrel defines an internal volume extending from the proximal end to the distal end of the barrel, said internal bore having a radius and being open at its proximal end. The distal tip defines a longitudinal passageway through which the medical product is expelled from the container. The distal tip is designed to receive a needle for injection of the medical product into an injection site. A plunger assembly is provided which comprises a plunger rod at the distal of which is arranged a plunger stopper, also simply called stopper or piston. The stopper is engaged in the internal reservoir of the barrel in a fluid tight manner so as to define, within the internal volume of the barrel, the volume of an internal reservoir defined between the stopper and the distal tip. The medical product is contained with this internal reservoir. The volume of the internal reservoir depends on the longitudinal position of the stopper in the barrel.

For injecting the medical product to patient, the syringe is first filled with the medical product in the internal reservoir. During injection, the user acts on the plunger rod to cause movement of the stopper inside the barrel in the distal direction, towards the distal tip. At the end of the injection, the stopper in the barrel is typically in the distal most position which corresponds to a position where the stopper abuts against the distal end of the barrel. The abutment of the stopper against of the distal end of the barrel is an easily recognizable position for the user when performing the injection.

The syringe typically has a maximum internal reservoir volume corresponding to the most proximal position for the stopper in the barrel. The maximum dose of medical product which can be injected to the patient is thus proportional to the stroke of the stopper between the most proximal safe position for the stopper and the most distal position of the stopper in the barrel.

In most cases, especially in the case of a prefilled syringes, the size of the syringe is adapted to the volume of medical product to be injected to the patient, in that its maximum internal reservoir matches the dose of medical product which is to be injected.

However, it is well-known that the dose of medical product which is to be injected may vary from one patient to the other, and/or may vary for a given patient, depending for example on the age of the patient, on the weight of the patient, on a medical condition of the patient, and/or depending on different steps of a treatment, et cetera. In many cases, it is up to the health professional in charge of performing the injection to define and set the dose of medical product which is needed for a given injection.

It is of course possible to have, for a given medical product, different syringes having a different initial quantity of the medical product filled into the syringe, so that the syringe used for performing the injection is selected based upon the dose to be injected. However, this this requires manufacturing, storing, handling and keeping track of different sets of syringes, of different volumes, to always have the syringe adapted to a given dose are defined for a given injection. This necessarily induces increased costs and/or what space for manufacturing, storing handling and keeping track of those different sets of syringes.

Alternatively, a minimal quantity of medical product may be expelled before the beginning of the injection, for priming the syringe and expelling any air bubbles.

Thus, there remains the need for providing to the health professionals a way to reliably inject a defined dose of medical product to a patient, at a minimized expenditure of resources.

### Summary

In its herein disclosed a plunger assembly for a syringe assembly comprising a syringe body, wherein the plunger assembly comprises:
- a plunger rod extending along a longitudinal axis and having a proximal end and a distal end;
- at the distal end of the plunger rod, a stopper mount portion for receiving a stopper.

The plunger assembly comprises a plunger stroke limiter. The position of the plunger stroke limiter with respect to the stopper mount portion is user-adjustable along the longitudinal axis, and the plunger stroke limiter is configured to abut against a syringe body abutment surface of the syringe body for at least one position of the plunger stroke limiter with respect to the stopper mount portion.

In some examples, the plunger stroke limiter is mounted on the plunger rod in one of at least two distinct positions with respect to the stopper mount portion along the longitudinal axis , and the position of plunger stroke limiter is user-adjustable to the other of the at least two distinct positions.

In some examples, the plunger stroke limiter comprises a ring which is co-axially mounted on the plunger rod.

In some examples, the plunger stroke limiter is mounted on the plunger rod via a helical thread.

In some examples, the plunger rod is formed of two parts comprising a proximal part and a distal part which can be connected one to the other according to at least two distinct positions with respect one to the other along the longitudinal axis, the stopper mount portion is formed on the proximal part, and the plunger stroke limiter is formed on the distal part. In some variants of such examples, the distal part and the proximal part of the plunger rod are telescopically connected. In some variants of such examples, the distal part and the proximal part of the plunger rod are telescopically connected via a helical thread. In some of examples and variants the proximal part of the plunger rod has a proximal end having comprises a radial plunger flange, and the radial plunger flange forms the plunger stroke limiter.

In some examples, the position of the plunger stroke limiter with respect to the stopper mount portion along the longitudinal axis is secured by a ratchet. In some variants of such examples, the ratchet mechanism is an asymmetric ratchet requiring a greater force to adjust a decrease of longitudinal distance between the plunger stroke limiter and the stopper mount portion than a force needed to adjust an increase of longitudinal distance between the plunger stroke limiter and the stopper mount portion, or a one-way irreversible ratchet allowing only an increase of the distance between the plunger stroke limiter and the stopper mount portion.

In some examples, the plunger stroke limiter comprises at least one lock which is adapted for locking the plunger stroke limiter on the syringe body.

In some examples, the plunger assembly further comprises a stopper mounted on the stopper mount portion of the plunger rod. In some variants of such examples, the stopper comprises at least one circular sealing rib having a sealing radius along a radial direction perpendicular to the longitudinal axis, and the plunger stroke limiter has a maximal radial extent with respect to the longitudinal axis which is greater than the sealing radius.

It is further disclosed a syringe assembly having a syringe body and plunger assembly having any of the features above. In some examples of such a syringe assembly, the syringe body comprises a syringe barrel which has a tubular shape along the longitudinal axis and an open proximal end defining a proximal facing end surface having an internal diameter around the longitudinal axis, wherein the maximum radial extent of the plunger stroke limiter is greater that than the internal diameter of the proximal facing end surface of the syringe barrel.

### Description of the drawings

[Fig. 1]: Figure 1 is a perspective view of a first example of a syringe assembly.
[Fig. 2]: Figure 2 is a schematic longitudinal section view of the syringe assembly of Figure 1, having a plunger assembly in a start position of the plunger assembly, with a plunger stroke limiter having a first position with respect to a stopper mount portion of the plunger.
[Fig. 3]: Figure 3 is a schematic section view of the syringe assembly of Fig. 2, with its plunger assembly in a first end position of the plunger assembly, with the plunger stroke limiter having the first position with respect to a stopper mount portion of the plunger.
[Fig. 4]: Figure 4 is a schematic section view of the syringe assembly of Figure 2, with its plunger assembly in the same start position of the plunger assembly, with the plunger stroke limiter having a second position with respect to a stopper mount portion of the plunger.
[Fig. 5]: Figure 5 is a schematic section view of the syringe assembly of Fig. 2, with its plunger assembly in a second end position of the plunger assembly, with the plunger stroke limiter having the second position with respect to a stopper mount portion of the plunger.
[Fig. 6]: Figure 6 is a perspective view of a second example of a syringe assembly.
[Fig. 7]: Figure 7 is a schematic cross-sectional view of the syringe assembly of Figure 6.
[Fig. 8]: Figure 8 is a detail of Figure 7.
[Fig. 9]: Figure 9 is a perspective view of a third example of a syringe assembly.
[Fig. 10]: Figure 10 is an exploded perspective view of a two-part plunger assembly for a fourth example of a syringe assembly.
[Fig. 11]: Figure 11 is a perspective view of the two parts plunger assembly of Figure 10, in a first relative position of the proximal part with respect to the distal part.
[Fig. 12] : Figure 12 is a perspective view of the two parts plunger assembly of Figure 10, in a second relative position of the proximal part with respect to the distal part
[Fig. 13]: Figure 13 is a schematic longitudinal section view of the syringe assembly of Figure 12, having the plunger assembly in a start position of the plunger assembly, with the two parts of the plunger assembly in the first relative position.
[Fig. 14] : Figure 14 is a schematic longitudinal section view of the syringe assembly of Figure 12, having the plunger assembly in a first end position of the plunger assembly, with the two parts of the plunger assembly in the first relative position
[Fig. 15] : Figure 15 is a schematic longitudinal section view of the syringe assembly of Figure 12, having the plunger assembly in the start position of the plunger assembly, with the two parts of the plunger assembly in a second relative position
[Fig. 16]: Figure 16 is a schematic longitudinal section view of the syringe assembly of Figure 12, having the plunger assembly in a second end position of the plunger assembly, with the two parts of the plunger assembly in the second relative position.

### Detailed description

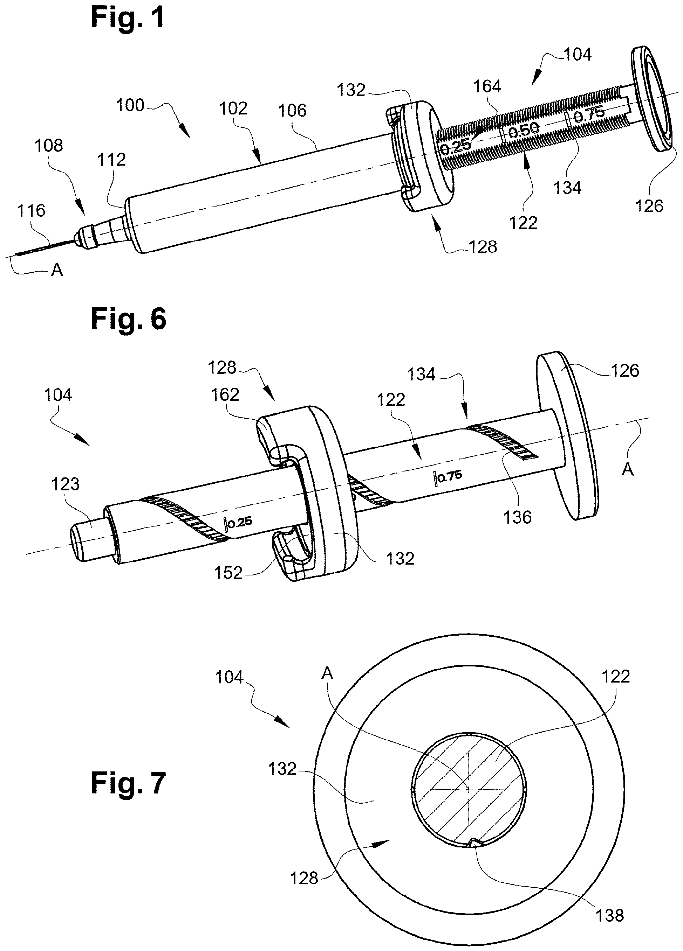

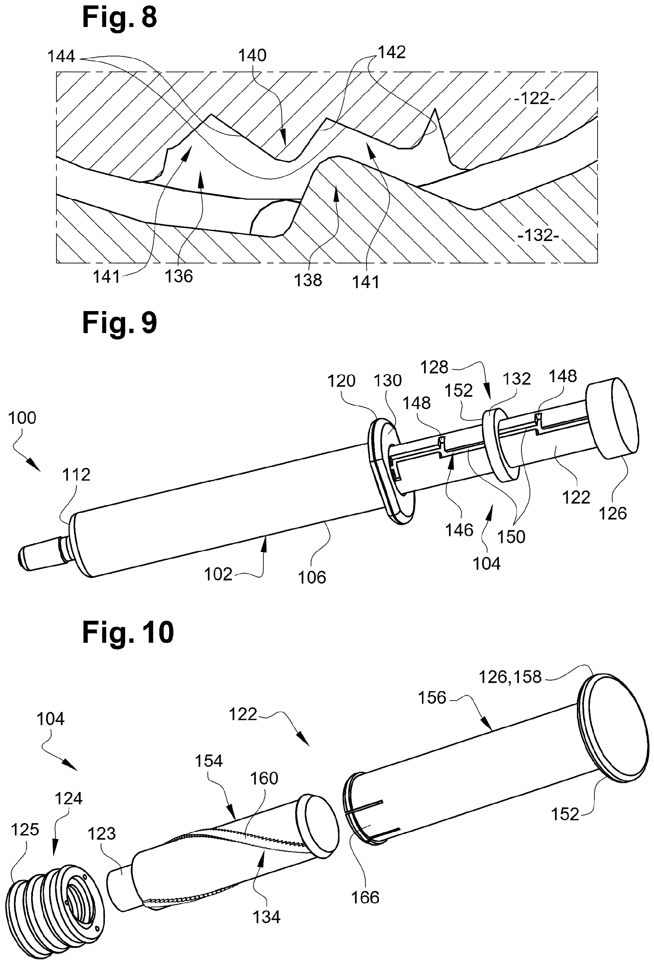

Four examples of a syringe assembly 100 will now be described. The first example is shown in Figures 1 to 5. A second example is shown on the Figures 6 to 8. A third example is shown on Figure 9. And a fourth example is shown on Figures 10 to 16. In all of these examples, the syringe assembly 100 comprises a syringe body 102 and a plunger assembly 104. In this text, where the syringe assembly 100 is designed to be handled by a user, typically a health professional during use, for instance during an injection procedure, the distal end of a component or of a device is to be understood as meaning the end furthest from the user's hand and the proximal end is to be understood as meaning the end closest to the user's hand. Likewise, in this application, the "distal direction" is to be understood as meaning the direction of injection along a longitudinal axis, and the "proximal direction" is to be understood as meaning the opposite direction to said direction of injection along the longitudinal axis A, that is to say the direction towards the user's hand.

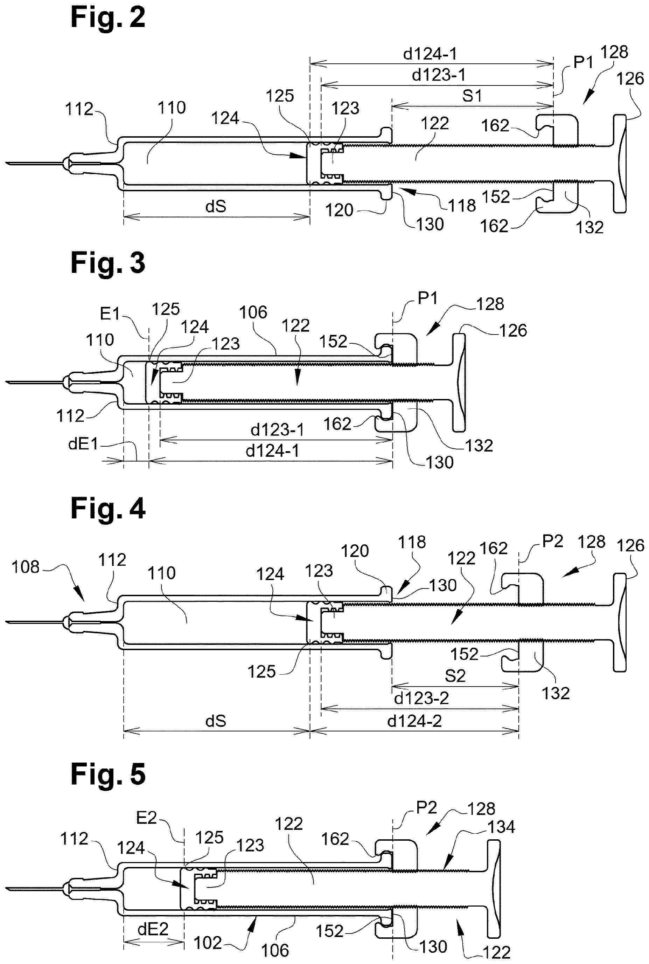

In all of the examples, syringe assembly 100 may be a prefilled or prefillable syringe assembly. The syringe body 102 includes a barrel 106, of tubular shape, having, along a longitudinal axis A, a proximal end, a distal end, and a distal tip 108 at its distal end. As more clearly visible in Figure 2, the barrel 106 defines an internal reservoir 110 which is designed for containing a medical product such as a drug which may be for example under liquid form. The barrel 106 may be made of a plastic or of a glass material. This barrel 106 has, at its distal end, a distal shoulder 112 provided with the distal tip 108 longitudinally protruding in the distal direction from said distal shoulder 112 along the longitudinal axis A. The distal tip 108 defines a longitudinal passageway in fluid communication with the reservoir 110 and is designed to be equipped with an injection needle 116 for injecting the medical product in an injection site of a patient. The injection needle 116 can be connected to the distal tip 108, for example via a Luer-type connection (not represented in drawings), e.g. a Luer-cone or Luer-lock connection in which the user attaches the needle at the time of injection, or via a staked or pre-attached needle in which the injection cannula is already pre-attached, for example via an adhesive. The barrel 106 further includes an open proximal end 118 which is bordered with a radial flange 120. The open proximal end 118 receives a plunger rod 122 for pushing a stopper 124 of the plunger rod assembly 104. As can be seen on the figures, the plunger rod 122 extends along the longitudinal axis A and has a proximal end and a distal end. The plunger rod 122 typically comprises, at its proximal end, a plunger flange 126 whose proximal facing surface forms a contact surface for the thumb of a user of the syringe assembly. At the distal end of the plunger rod 122, a stopper mount portion 123 is provided for receiving the stopper 124.

In all the shown examples, the stopper 124 is a part which is separate from the plunger rod 122. Therefore, in such a case, the stopper mount portion 123 is configured to allow the attachment of the stopper 124. The respective design of the stopper mount portion 123 and of the stopper 124 can be according to any design known to the skilled person in the art. The stopper mount portion 123 can be a screw portion designed to match a threaded hollow aperture of the stopper 124 to allow the screwing of the stopper 124 on the stopper mount portion of 123. In other designs, the stopper mount portion 123 can be designed as a stepped segment allowing the forceful mounting of the stopper 124 on the stopper mount portion 123, the respective design of the stopper mount portion 123 and of the stopper 124 preventing unintentional dismounting of the stopper 124. In use of the syringe assembly, the stopper 124 is mounted on the stopper mount portion 123 of the plunger rod 122 and can be considered to have, at all times, a fixed position with respect to the stopper mount portion 123.

The stopper 124 and a distal portion of the plunger rod 122 are, in a use configuration of the syringe assembly 100, received inside the syringe barrel 106. The stopper 124 is able to slide longitudinally in a fluid-tight manner inside the syringe barrel 106 under the action of the plunger rod 122 to which it is connected.

Preferably, the syringe barrel 106 has constant diameter along a useful length between a most proximal position and a most distal position of the stopper 124 in the syringe barrel 106.

The stopper 124 has at least one peripheral sealing surface, such as a circular sealing rib 125, which peripherally engages in a fluid tight manner an inner cylindrical surface of the syringe barrel 106. The sealing surface 125 of the stopper 124 is preferably elastic to provide fluid tightness. The sealing surface is typically made of an elastomer material.

The sealing surface 125 has a sealing radius, along a radial direction perpendicular to the longitudinal axis, which is slightly superior to the radius of the syringe barrel 106, i.e. slightly superior to the half diameter of the syringe barrel 106, so that when the stopper 124 is engaged inside the barrel, the sealing surface achieves the fluid tight contact between the stopper 124 and the syringe barrel 106, while still allowing longitudinal movement of the stopper 124 inside the syringe barrel 106. The stopper 124 is thus the portion of the plunger assembly 104 which delimits in a fluid-tight manner the volume of the reservoir 110 of medical product inside the syringe barrel 106, said volume being dependent on the position of the stopper 124 inside the syringe barrel 106 along the longitudinal axis A.

When the stopper 124 is pushed along the distal direction by the plunger rod 122, it causes the expulsion of the medical product from the reservoir 110 to the injection site via the longitudinal passageway through the distal tip 108 and the injection needle 116.

Typically, when using a syringe assembly 100 to inject a medical product during an injection procedure, the syringe assembly is first configured in a start configuration where the plunger assembly 122 has a start position with respect to the syringe barrel 106. In that start position of the plunger assembly 122, the stopper 124 occupies, inside the syringe barrel 106, a start position with respect to the syringe barrel. The position of the stopper 124 inside the syringe barrel 106 may be defined arbitrarily by the position of the most distal peripheral sealing surface 125 of the stopper with respect to the syringe barrel 106, for example with respect to the distal shoulder 112. Alternatively, such position could have been defined by another part of the stopper 124, for example the most distal or the most proximal part of the stopper 124. In the start configuration, the reservoir 110 delimited by the stopper 124 inside the syringe barrel 106 is filled with the medical product. Such start configuration of the syringe assembly 100, and thus such start position of the plunger assembly 122 and such start position of the stopper 124 are shown, with respect to this first example of the syringe assembly 100, in Figure 2 and in Figure 4.

Typically, in the start position, the volume of the reservoir 110 containing the medical product, which is defined by the start position of stopper 124, and thus defined by the position of the stopper mount portion 123 of the plunger rod 122, is maximum. This start position is determined at the time of the design of the syringe assembly, taking into consideration, amongst other things, the stability of the plunger assembly 104 with respect to the syringe barrel 106. The entirety of that volume is, at the start of the injection procedure, filled with the medical product.

The start position of the plunger assembly 104 can be identified by a distance dS, along the longitudinal axis A, between a reference point chosen on the stopper 124 or on the stopper mount portion 123 and a reference point chosen on the syringe body 102, such as for example the distal shoulder 112 of the syringe body 102.

When performing the injection, the user pushes the plunger assembly 122 in the distal direction until the plunger assembly 122 reaches an end position with respect to the syringe barrel 106. In that end position of the plunger assembly, which corresponds to an end configuration of the syringe assembly 100, the stopper 124 occupies, inside the syringe barrel 106, an end position with respect to the syringe barrel 106. Such end configuration of the syringe assembly 100, and thus such end position of the plunger assembly 122 and such end position of the stopper 124 are shown, with respect to this first example of the syringe assembly 100, in Figure 3 and in Figure 5.

Thus, during such injection, a volume of medical product has been expelled from the syringe assembly 100 and injected to the patient. The injected volume is proportional to the stroke of the stopper 124 between its start position and its end position, i.e. the distance travelled by the stopper 124, along the longitudinal axis A, with respect to the syringe barrel 106, between the start position of the stopper 124 and the end position of the stopper 124.

In the various examples of syringe assembly 100 which are represented in the figures and which will be disclosed in detail below, the plunger assembly 104 comprises a plunger stroke limiter 128, 158. The position of the plunger stroke limiter 128, 158 with respect to the stopper mount portion 123 is user-adjustable along the longitudinal axis A. The plunger stroke limiter 128, 158 is configured to abut against a syringe body abutment surface 130 of the syringe body 102 for at least one position of the plunger stroke limiter 128, 158 with respect to the stopper mount portion 123.

Because the stopper 124 has a fixed position with respect to the stopper mount portion 123, the position of the plunger stroke limiter 128, 158 with the respect to the stopper 124 is user adjustable along the longitudinal axis A. The position of the plunger stroke limiter 128, 158 with respect to the stopper mount portion 123 can be defined as a distance, along the longitudinal axis, between the plunger stroke limiter 128, 158 and the stopper mount portion 123. When a stopper 124 is mounted on the stopper mount portion 123, the position of the plunger stroke limiter 128, 158 with respect to the stopper mount portion 123 can alternatively be defined by the distance, along the longitudinal axis, between the plunger stroke limiter 128, 158 and the stopper 124. The position of the plunger stroke limiter 128, 158 with respect to the stopper mount portion 123 and/or with respect to the stopper 124 does not vary during an injection procedure.

In the first example of a plunger assembly 104 having a plunger stroke limiter 128, as well as in the second example of Figures 6 to 9, and in the third example of Figure 10, the plunger rod 122 is a rigid element having a fixed length from its distal end to its proximal end, i.e. in those examples from the plunger flange 126 to the stopper mount portion 123, and the plunger stroke limiter 128 is mounted on the plunger rod 122 in one of at least two distinct positions with respect to the stopper mount portion 123 along the longitudinal axis A. The position of plunger stroke limiter 128 is user-adjustable to the other of the at least two distinct position.

As expressly represented for the first example of Figures 1 to 5, the plunger stroke limiter 128 can thus be mounted in a first position P1 on the plunger rod 122, with respect to the stopper mount portion 123, as shown in Figures 2 and 3, or in at least a second position P2 on the plunger rod 122, with respect to the stopper mount portion 123, as shown in Figures 4 and 5.

In the first position P1 of the plunger stroke limiter 128 with respect to the stopper mount portion 123, the plunger stroke limiter 128 is at a first distance d123-1 of the stopper mount portion 123 and at a first distance d124-1 with respect to the stopper 124. In the second position P2 of the plunger stroke limiter 128 with respect to the stopper mount portion 123, the plunger stroke limiter 128 is at a second distance d123-2 of the stopper mount portion 123 and at a second distance d124-2 with respect to the stopper 124. In the example, the reference points used for the respective positions of the stopper mount portion 123 and of the stopper 124 are not coincident, but it is to be understood that any reference point could be chosen on the stopper mount portion 123 and on stopper 124 as being the reference point for the respective positions of the stopper mount portion 123 and of the stopper 124. Once chosen, the reference point is kept the same. Similarly, any reference point can be chosen on the plunger stroke limiter 128 as being the reference point used for determining the position the plunger stroke limiter 128. In the example of Figures 2 to 5, the reference point used for determining the position the plunger stroke limiter 128 is a distal facing stroke limiting surface 152 of the plunger stroke limiter 128.

In the first example of Figures 1 to 5, in the second example of Figures 6 to 9, and in the third example of Figure 10, the plunger stroke limiter comprises a ring 132 which is annular around the longitudinal axis A and which is co-axially mounted on the plunger rod 122, preferably on a cylindrical segment of the plunger rod 122 between the proximal end and the distal end of the plunger rod 122. The cylindrical segment of the plunger rod 122, which is cylindrical around the longitudinal axis A, may extend the full length of the plunger rod from its proximal end to its distal end, or may extend only along a portion of that length. In the shown first second and third examples, the ring 132 is an annular ring extending at 360° around the longitudinal axis on the cylindrical segment of the plunger rod 122.

In the first and second examples, the plunger stroke limiter 128 is mounted on the plunger rod 122 via a helical thread 134. The helical thread 134 induces a direct and positive correlation between an angular position of the plunger stroke limiter 128 around the longitudinal axis A and the longitudinal position of said plunger stroke limiter 128 on the plunger rod 122. Thanks to the helical thread 134, the position of the plunger stroke limiter 128 on the plunger rod 122 can be easily adjusted by the end-user, by rotating the ring 132 around the longitudinal axis to the extent necessary to displace the plunger stroke limiter to a desired longitudinal position on the plunger rod 122, thus with respect to the stopper mount portion 123.

In the first example of Figures 1 to 5, the helical thread 134 has a reduced pitch, corresponding to the small lead angle of the thread. The plunger rod 122 has a thus a conventional external helical thread on its outer cylindrical surface, extending from proximal thread end to a distal thread end on the plunger rod 122. Correspondingly, the ring 132 of the plunger stroke limiter 128 has a conventional internal helical thread on its inner cylindrical surface. The lead angle of the helical thread 134 is the angle formed by the inclination of the helix of the thread with a plane that is perpendicular to the longitudinal axis A. For example, the lead angle of the thread is comprised between 0.5° and 15° of angle, preferably comprise between 1° and 5° of angle. With such a small lead angle, any relative force applied along the longitudinal axis A on the ring 132 and/or on the plunger rod 122 will not cause any relative rotation between the ring 132 and the plunger rod 122, thus will not cause any relative longitudinal displacement between the ring 132 and the plunger rod 122. Thus, in this first example, the helical thread 134 is a smooth thread having no indexing means. Thus, the plunger stroke limiter can be positioned at any of an infinite number of longitudinal positions on the plunger rod 122 between the proximal thread end and the distal thread end.

In the second example of Figures 6 to 9, the helical thread 134 has a large pitch, corresponding to a larger lead angle of the thread. For example the lead angle of the thread is comprised between 20° of angle and 60° of angle. With such a large lead angle, a quicker adjustment of the position of the plunger stroke limiter is possible, with a limited amount of rotation of the ring 132. In the example, the helical thread 134 extends along substantially the whole length of the plunger rod 122 between the proximal thread end and the distal thread end, and the longitudinal position of the plunger stroke limiter can be shifted between the proximal thread end and the distal thread end with a total rotation of the ring 132 which is comprised between two and three complete turns around the longitudinal axis A.

In the second example of Figures 6 to 9, the helical thread 134 comprises a helical groove 136 formed on the outer cylindrical surface of the plunger rod 122. As more clearly visible on Figures 7 and 8, the ring 132 of the plunger stroke limiter 128 has, on a cylindrical internal surface, a radially inwardly oriented prong 138 which projects radially inwardly inside the helical groove 136 and which interacts with said helical groove 136 to form the helical thread connection between the plunger stroke limiter 128 and the plunger rod 122 by which a longitudinal position of the plunger stroke limiter 128 corresponds directly and positively with an angular position around the longitudinal axis A.

In this example, the position of the plunger stroke limiter 128 with respect to the plunger rod 122 along the longitudinal axis may be secured by indexing means, such as for example a ratchet mechanism. Whilst such indexing means could be provided also in the first example, the indexing means are here a particularly useful because the large lead angle of the helical thread 134 allows for forces applied along the longitudinal axis A on the ring 132 and/or on the plunger rod 122 to possibly cause a relative rotation between the ring 132 and the plunger rod 122, which would cause a corresponding relative longitudinal displacement between the ring 132 and the plunger rod 122. The indexing means prevents any unwanted displacement of the plunger stroke limiter 128 with respect to the plunger rod 122, at least for the forces normally applied to the syringe assembly 100 during normal use.

In the example of Figures 6 to 9, the ratchet mechanism is a helical ratchet having a ratchet in the form of a helix around the longitudinal axis A. The ratchet mechanism comprises ratchet teeth 140 formed in the helical groove 136, in this example on the bottom surface of the helical groove 136. Two consecutive the ratchet teeth 140 are separated, along the helical extension of the helical groove 136 by a recess 141. The radially inwardly oriented prong 138 of the plunger stroke limiter 128 cooperates with the ratchet teeth 140 so as to automatically lock when received in a recess 141 between two ratchet teeth 140, while allowing the prong 138 to move, under the application of sufficient force, over one of the said ratchet teeth 140 to the next recess 141 and to automatically lock in that next recess 141. The ratchet mechanism thus determines a finite number of locking positions corresponding each to a recess 141 between two ratchet teeth 140. In this example, the ratchet teeth 140 protrude radially from the bottom surface of the helical groove 136. However, the ratchet teeth 140 could be configured to protrude perpendicularly from one or both of the lateral helical side surfaces of the helical groove 136.

Advantageously, the ratchet mechanism can be configured as an asymmetric ratchet. In such an asymmetric ratchet, a greater force is required to adjust a decrease of longitudinal distance between the plunger stroke limiter 128 and the stopper mount portion 123 than a force needed to adjust an increase of longitudinal distance between the plunger stroke limiter 128 and the stopper mount portion 123. As can be seen in Figure 8, the ratchet teeth 140 are each defined between two opposites side surfaces 142, 144 which have a different angle with respect to the relative displacement of the prong 138 and of the ratchet teeth 140 along the helical extension of the helical thread 134. The prong 138 of the plunger stroke limiter 128 may have a corresponding asymmetric profile. By proper selection of these angles, an asymmetric effect can be obtained. In some cases, an asymmetric ratchet may be a one-way irreversible ratchet allowing only an increase of the distance between the plunger stroke limiter 128 and the stopper mount portion 123, for example by designing the ratchet teeth 140 with a side surface forming an angle of approximately 90° with respect to the relative displacement of the prong 138 and of the ratchet teeth 140.

In the second example of Figures 6 to 9, the ratchet mechanism defines a relatively high finite number of discrete locked positions of the plunger stroke limiter 128 on the plunger rod 122, thus with respect to the stopper mount portion 123 and to the stopper 124, along the longitudinal axis A, with for example 50 or more discrete locked positions, or 100 or more discrete locked positions. This allows for a fine adjustment of the position of the plunger stroke limiter 128 along the plunger rod 122.

In the third example of Figures 6 to 9, the plunger assembly 104 is provided with a plunger stroke limiter 128 which can be positioned by a user and locked in only a limited amount of discrete locked positions on the plunger rod 122, thus with respect to the stopper mount portion 123, along the longitudinal axis A, for example 10 or less discrete locked positions of the plunger stroke limiter 128 with the respect to the plunger rod 122 along the longitudinal axis A.

In this example, the plunger rod 122 exhibits, in an outer cylindrical surface thereof, a segmented groove 146 which has a limited finite number of transverse segments 148 of the groove, which extend perpendicularly to the longitudinal axis and which are each connected one to the other by longitudinal segments 150 of the groove. The transverse segments 148 correspond each to one discrete locked longitudinal position of the plunger stroke limiter 128 with respect to the plunger rod 122, thus with respect to the stopper mount portion 123. They are spaced apart at predefined positions along the longitudinal axis A. The plunger stroke limiter 128 comprises an annular ring 132 which is equipped with a radially inwardly oriented prong (not visible in the drawings) which is received in the segmented groove 146. To allow for the longitudinally positioning of the plunger stroke limiter 128, the annular ring 132 is rotated around the longitudinal axis A so as to align the prong 138 with a longitudinal segment 150, to allow longitudinal movement of the plunger stroke limiter 128. To the lock plunger stroke limiter 128 in a predefined position, its prong is engaged into a corresponding one of the transverse segments 148.

In all of the examples described above, the plunger stroke limiter 128 has a maximal radial extent with respect to the longitudinal axis A which is greater than the sealing radius. In other words, the plunger stroke limiter 128 has a maximal radical extent with respect to the longitudinal axis A which is greater than the internal radius of the syringe barrel 106. As a consequence, depending on the position of the plunger stroke limiter 128 on the plunger rod 122, thus with respect to the stopper mount portion 123, the plunger stroke limiter 128 may interfere with the syringe body 102 to limit the ability of the plunger assembly 104 to be pushed inside the syringe barrel 106 in the distal direction. Typically, the plunger stroke limiter 128 comprises a distally facing plunger stroke limiting surface 152 which extends radially further than the sealing radius of the stopper 124 along the radial direction. In the first, second and third examples, this distally facing plunger/limiting surface 152 is a distally facing surface of the ring 132, which is configured to interfere with the proximal end of the syringe barrel 106, which, in the examples, is a proximally facing surface 130 of the radial flange 120 of the syringe barrel 106. More particularly, the syringe barrel 106 has a tubular shape along the longitudinal axis A and an open proximal end 118 defining a proximal facing end surface 130 having an internal diameter around the longitudinal axis A. The maximum radial extent of the plunger stroke limiter 128 is greater than the internal diameter of the proximal facing end surface 130 of the syringe barrel 106 so that an interference occurs between the plunger stroke limiter 128 and the syringe barrel 106 when the former reaches the longitudinal position of the latter.

As visible when comparing Figures 2 to 5, the first position P1 of the plunger stroke limiter 128 with respect to the stopper mount portion 123 corresponds to the first end position E1 of the stopper 124 in the syringe barrel 106. In this example, the first end position E1 of the stopper 124 is reached when the plunger stroke limiter 128 abuts against the syringe body abutment surface 130, as shown for example in Figure 3. When the plunger stroke limiter 128 is set to its first position, it allows, for the plunger assembly 104 and especially for the stopper 124, a first stroke S1 between its start position and its end position. In this example, the first stroke S1 is the distance, along the longitudinal axis, between the distally facing plunger stroke limiting surface 152 of the plunger stroke limiter 128 and the syringe body abutment surface 130 of the syringe body 102, when the plunger stroke limiter 128 is set to its first position P1 with respect to the stopper mount portion 123 and is in its start position with respect to the syringe body 102. However, it is to be noted that, for certain positions of the plunger stroke limiter 128 with respect to the to the stopper mount portion 123, the first end position E1 of the stopper 124 may be reached when the stopper 124 abuts against the distal shoulder 112, without the plunger stroke limiter 128 abutting against the syringe body abutment surface 130, as will be exemplified in the fourth example below.

The second position P2 of the plunger stroke limiter 128 with respect to the stopper mount portion 123 corresponds to a second end position E2 of the stopper 124 in the syringe barrel 106 when the plunger stroke limiter 128 abuts against the syringe body abutment surface 130, as shown for example in Figure 5. When the plunger stroke limiter 128 is set to its second position P2, it allows, for the plunger assembly 104 and especially for the stopper 124, a second stroke S2 between its start position and its end position E2. The second stroke S2 is the distance, along the longitudinal axis, between the distally facing plunger stroke limiting surface 152 of the plunger stroke limiter 128 and the syringe body abutment surface 130 of the syringe body 102, when the plunger stroke limiter 128 is set to its second position P2 with respect to the stopper mount portion 123 and is in its start position with respect to the syringe body 102.

In the example, the second position P2 of the plunger stroke limiter 128 is longitudinally closer to the stopper mount portion 123 of the plunger rod 122 thus closer the stopper 124, than the first position A1. As a consequence, in the end configuration of the syringe assembly 100, the second end position E2 of the stopper 124, as shown on Figure 5, is further away from distal shoulder 112 of the syringe barrel 106 than the first end position E1 of the stopper 124 shown in Figure 3.

For a given start position of the plunger assembly 104, more precisely for a given start position of the stopper 124, which is shown to be identical in Figures 2 and 4, the difference in longitudinally position of the plunger stroke limiter 128 on the plunger rod 122 between the first configuration and the second configuration of the plunger assembly 104 translates into the same difference between the stroke S1, S2 of the plunger assembly, particularly of the stopper 124, between its start position and its end position E1, E2, in the first and second configuration respectively.

In this example, for a given start position of the plunger assembly 104, the stroke S1, S2 of the plunger assembly 104, more particularly of the stopper 124, between the start position and the end position E1, E2, is equal to the distance, in the start position of the plunger assembly 104, between the distantly facing plunger/limiting surface 152 and the proximal end of the syringe barrel 106.

As a result of this difference in stroke, a different volume of medical product will be injected during the injection if the plunger assembly 104 is displaced from the start position to the end position which is limited and determined by the plunger stroke limiter 128.

In the first and second examples of the plunger assembly 104 as shown respectively in Figures 1 to 5 and in the Figures 6 to 9, the plunger stroke limiter 128 may advantageously comprise at least one locking portion which is adapted for locking the plunger stroke limiter 128 on the syringe barrel 106 when the plunger assembly 104 reaches the end position with respect to the syringe barrel 106.

As discussed above, the syringe barrel 106 has a radial end flange 120 at its open proximal end 118, and the at least one locking portion of the plunger stroke limiter is adapted for locking the plunger stroke limiter 128 on the radial end flange 120 of the syringe barrel 106.

In the shown examples, the locking portion of the plunger stroke limiter 128 comprises two locks which, in the examples, comprise each one harpoon 162. Each harpoon 162 extends distally along the longitudinal direction from the ring 132, from diametrically opposite locations of the ring 132 with respect to the longitudinal axis A. Each harpoon 162 extends longitudinally in the distal direction beyond the distally facing plunger stroke limiting surface of the ring 132, to engage and lock on the radial flange 120 of the syringe barrel 106. Each harpoon 162 has a distal extremity forming a hook which cooks beyond the radial flange 120 and thus impedes any retraction of the plunger assembly 104 in the proximal direction with respect to the syringe barrel 106. Each harpoon 162 exhibits sufficient flexibility along the radial direction for its hook to elastically deform when engaging on the radial flange 120. With such a locking portion, the unintended retraction of the plunger assembly 104 is avoided once it has reached its in position at the end of the injection, whatever the end positioned is, as defined by the longitudinal position of the plunger stroke limiter 128 on the plunger rod 122 with respect to the stopper mount portion 123.

In the first and second examples of the plunger assembly 104, the plunger assembly 104 has a graduation scale 164 with a moving marker associated to the distance between the plunger stroke limiter 128 and the stopper mount portion 123. In the example, the graduation scale comprises graduation indicia marked on the plunger rod 122, and the moving marker is formed by plunger stroke limiter 128. The graduation scale 164 indicates a maximum injectable volume or maximum injectable volume ratio associated with the distance between the distance between the plunger stroke limiter 128 and the stopper mount portion 123.

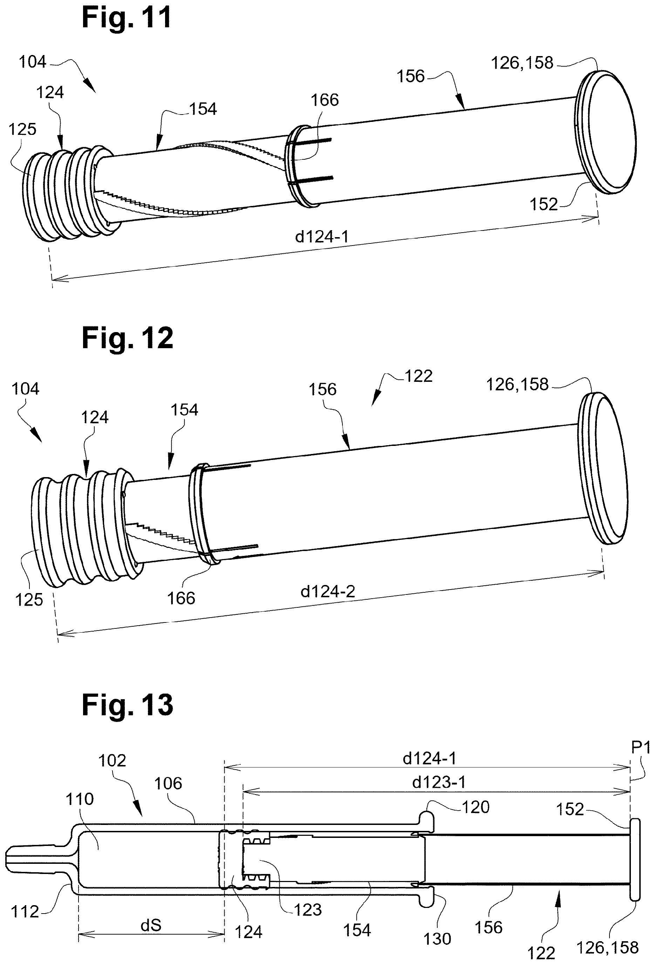

In the fourth example which is shown on Figures 10 to 16, the plunger rod 122 is formed of two parts comprising a distal part 154 and a proximal part 156, which are clearly visible on Figure 10, and which can be connected one to the other according to at least two distinct positions with respect one to the other along the longitudinal axis A, said two positions being exemplified respectively at Figure 11 and Figure 12. In this example the total length of the plunger rod 122 is adjustable.

The stopper mount portion 123 is formed on the distal end of the distal part 154, at a fixed location thereof, and the plunger stroke limiter 158 is formed on the proximal part 156, at a fixed location thereof. The stopper mount portion 123 can be configured as described above for the other examples.

In the fourth example of Figures 10 to 16, the plunger stroke limiter 158 is formed by the plunger flange 126. The proximal part 156 of the plunger rod 122 has a proximal end having the radial plunger flange 126 which forms the plunger stroke limiter 158. The radial plunger flange 126 has a maximal radial extent with respect to the longitudinal axis A which is greater than the sealing radius of the stopper 124 mounted on the stopper mount portion 123. The radial plunger flange 126 comprises a distally facing surface which forms a plunger stroke limiting surface 152 which extends radially further than the sealing radius of the stopper 124 along the radial direction.

In this example, the position of the plunger stroke limiter 158 with respect to the stopper mount portion 1553 is user-adjustable along the longitudinal axis A by adjusting the total length of the plunger rod 122.

The distal part 154 of the plunger rod 122 and the proximal part 156 of the plunger rod 122 are telescopically connected one to the other and they are configured to be set one to the other according to at least two distinct positions with respect one to the other along the longitudinal axis A. The plunger rod 122 may thus be adjusted to exhibit a fully extended configuration, with a maximum total length, as shown in Figures 11, 13 and 14, to exhibit a fully contracted configuration, with a minimum total length. In an example, the plunger rod 122 may be adjusted to exhibit a partially contracted configuration, with an intermediate total length as shown in Figures 12, 15 and 16. In the partially contracted and in the fully contracted configurations of the plunger rod 122, one of the distal or proximal parts of the plunger rod 122 is at least partially contained in the other of the distal or proximal parts of the plunger rod 122. In the shown example, the proximal part 126 is tubular, and, in the partially contracted and in the fully contracted configurations of the plunger rod 122, the distal part 154 of the plunger rod 122 is at least partially contained in the proximal part 156 of the plunger rod 122.

When the distal part 154 of the plunger rod 122 and the proximal part 156 of the plunger rod 122 are set according to a first position with respect one to the other along the longitudinal axis A, as shown in Figures 11, 13 and 14, the position of the plunger stroke limiter 158 is set to a first position P1 with respect to the stopper mount portion 123, and thus with respect to the stopper 124.

When the distal part 154 of the plunger rod 122 and the proximal part 156 of the plunger rod 122 are set according to a second position with respect one to the other along the longitudinal axis A, as shown in Figures 12, 15 and 16, the position of the plunger stroke limiter 158 is set to a second position P2 with respect to the stopper mount portion 123, and thus with respect to the stopper 124.

In the first position P1 of the plunger stroke limiter 158 with respect to the stopper mount portion 123, the plunger stroke limiter 158 is at a first distance d 123-1 of the stopper mount portion 123 and at a first distance d124-1 respect to the stopper 124. In the second position P2 of the plunger stroke limiter 158 with respect to the stopper mount portion 123, the plunger stroke limiter 158 is at a second distance d123-2 of the stopper mount portion 123 and at a second distance d124-2 respect to the stopper 124. In the example, the reference points used for the respective positions of the stopper mount portion 123 and of the stopper 124 are not coincident, but it is to be understood that any reference point could be chosen on the stopper mount portion 123 and on stopper 124 as being the reference point for the respective positions of the stopper mount portion 123 and of the stopper 124. Once chosen, the reference point is kept the same. Similarly, any reference point can be chosen on the plunger stroke limiter 158 as being the reference point used for determining the position the plunger stroke limiter 158. In the example of Figures 13 to 16, the reference point used for determining the position the plunger stroke limiter 158 is a distal facing stroke limiting surface 152 of the plunger stroke limiter 158.

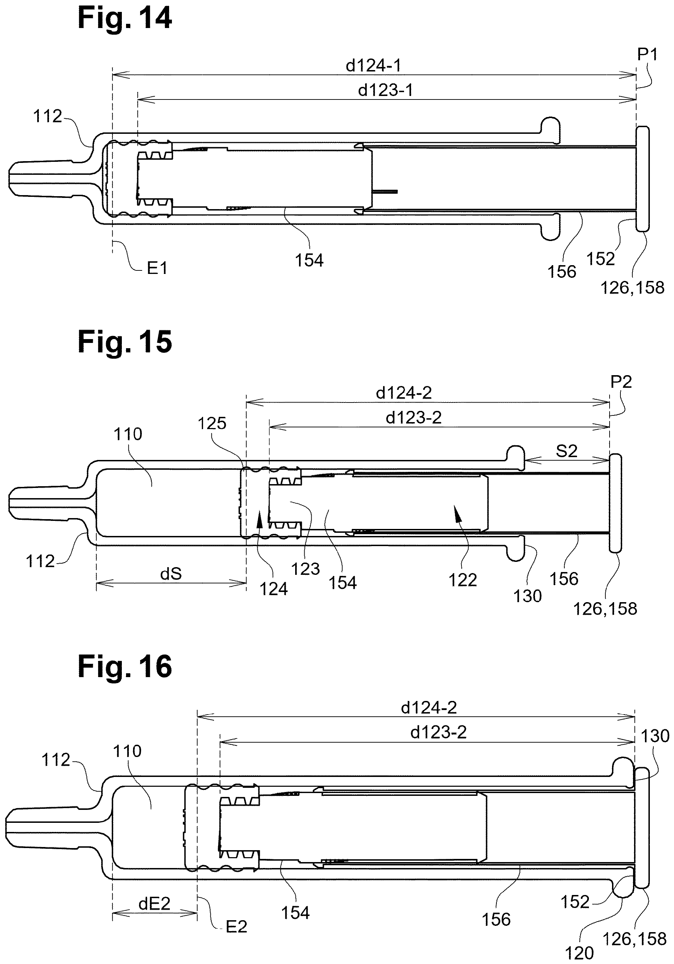

As visible when comparing Figures 13 to 16, the first position P1 of the plunger stroke limiter 158 with respect to the stopper mount portion 123 corresponds to the first end position E1 of the stopper 124 in the syringe barrel 106. In this example, the first end position E1 of the stopper 124 is reached when the stopper 124 abuts against the distal shoulder 112 of the syringe barrel, without the plunger stroke limiter 158 abutting against the syringe body 102, as shown for example in Figure 14. In this example of a first position P1 of the plunger stroke limiter 158 with respect to the stopper mount portion 123, the plunger stroke limiter 158 does not limit the stroke of the piston assembly. When the plunger stroke limiter 158 is set to its first position by adjusting the total length of the plunger rod 122, it allows, for the plunger assembly 104 and especially for the stopper 124, a first stroke S1 between its start position and its end position. In the example of Fig. 14, the plunger rod is extended to its maximum total length. In this example, the first stroke S1 is the maximum stroke available for the syringe assembly 100, for a given start position, and is the distance, along the longitudinal axis A, between the stopper 124 and the distal shoulder 112 of the syringe body 102 when the stopper is in the start position. However, it is to be noted that, for certain positions of the plunger stroke limiter 158 with respect to the to the stopper mount portion 123, the first end position E1 of the stopper 124 may be reached when the plunger stroke limiter 158 abuts against the syringe body.

The second position P2 of the plunger stroke limiter 158 with respect to the stopper mount portion 123 corresponds to a second end position E2 of the stopper 124 in the syringe barrel 106 when the plunger stroke limiter 158 abuts against the syringe body abutment surface 130, as shown for example in Figure 16. The second end position E2 of the stopper 124 is different from the first end position E1. When the plunger stroke limiter 158 is set to its second position P2 by adjusting the total length of the plunger rod 122, here an intermediate length or a minimal length, it allows, for the plunger assembly 104 and especially for the stopper 124, a second stroke S2 between its start position and its end position E2. The second stroke S2 is the distance, along the longitudinal axis, between the distally facing plunger stroke limiting surface 152 of the plunger stroke limiter 158 and the syringe body abutment surface 130 of the syringe body 102, when the plunger stroke limiter 158 is set to its second position P2 with respect to the stopper mount portion 123 and is in its start position with respect to the syringe body 102.

In the example, the second position P2 of the plunger stroke limiter 158 is longitudinally closer to the stopper mount portion 123 of the plunger rod 122 thus closer the stopper 124, than the first position A1. As a consequence, in the end configuration of the syringe assembly 100, the second end position E2 of the stopper 124, as shown on Figure 16, is further away from distal shoulder 112 of the syringe barrel 106(see distance dE2 in figure 15) than the first end position E1 of the stopper 124 shown in Figure 14, where the stopper abuts against the distal shoulder E1 in the shown example.

For a given start position of the plunger assembly 104, more precisely for a given start position of the stopper 124, which is shown to be identical in Figures 13 and 15, the difference in longitudinal position of the plunger stroke limiter 158 with respect to the piston mount portion 123, which is reflects the difference in total length of the piston rod 122 between the first configuration and the second configuration of the plunger assembly 104, translates into the same difference between the stroke S1, S2 of the plunger assembly, particularly of the stopper 124, between its start position and its end position E1, E2, in the first and second configuration respectively.

As a result of this difference in stroke, a different volume of medical product will be injected during the injection if the plunger assembly 104 is displaced from the start position to the end position which is limited and determined by the plunger stroke limiter 158.

In the example of Figures 10 to 16 the distal part 154 and the proximal part 156 of the plunger rod 154 are telescopically connected via a helical thread 134. The helical thread 134 induces a direct and positive correlation between the relative angular positions of the distal and proximal parts of the plunger rod 122 around the longitudinal axis A and the relative longitudinal positions of the distal and proximal parts of the plunger rod 122. Thanks to the helical thread 134, the position of the plunger stroke limiter 158, which is carried by the proximal part 156 of the plunger rod 122, with respect to the stopper mount portion 123, which is carried by the distal part 154, can be easily adjusted by a relative rotation, by the end-user, of the two parts of the plunger rod 122 around the longitudinal axis to the extent necessary to displace the plunger stroke limiter 158 to a desired longitudinal position with respect to the stopper 124, by a corresponding adjustment of the total length of the plunger rod 122.

In the shown example, the helical thread 134 is formed of a helical rail 160 formed on an outer cylindrical surface of the distal part 154 of the plunger rod 122. The proximal part 156 of the plunger rod 122 has, at its distal end, a flexible pawl 166 which cooperates with the helical rail 160 to form a helical thread connection between the two parts of the plunger rod 122.

Preferably, as in the previous examples, the position of the plunger stroke limiter 126, 156 with respect to the stopper mount portion 123 along the longitudinal axis A is secured by a ratchet mechanism. In the shown example, the ratchet mechanism is arranged between the distal part 154 and the proximal part 156 of the plunger rod 122. The ratchet mechanism may be designed in a same or similar way as described above. It may be an asymmetric ratchet mechanism.

In all the examples, the plunger assembly 104 can be used to from a syringe assembly 100 which can filled with a maximal volume of the medical product. For example the syringe assembly can be used as a prefilled assembly which is provided to the end-user, i.e. the medical professional which is to perform the injection, with the plunger assembly in a start configuration where the volume of the reservoir 110 containing the medical product is maximum. This volume corresponds to the maximum dose of medical product which may be injected with the syringe assembly 100. Preferably, the position of the plunger stroke limiter 128, 158 with respect to the stopper mount portion 123 may be adjusted to at least a first position where the plunger stroke limiter 128, 158 does not limit the stroke of the piston assembly, so that, in that first position of the plunger stroke limiter 128, 158, the maximum dose of medical product which may be injected with the syringe assembly 100. In that first position of the plunger stroke limiter 128, 158, the plunger stroke limiter 128, 158 does not abut against the barrel abutment surface of the syringe barrel 106, or barely abuts against the barrel abutment surface of the syringe barrel 106 until the stopper 124 has reached a maximum end position where the volume of the reservoir containing the medical product is minimal. However, the position of the plunger stroke limiter 128, 158 with respect to the stopper mount portion 123 may be adjusted by the end-user to at least a second position where the plunger stroke limiter 128, 158 does limit the stroke of the piston assembly. In that second position of the plunger stroke limiter 128, 158, only a fraction of the maximum dose of medical product may be injected with the syringe assembly 100. In that second position of the plunger stroke limiter 128, 158, the plunger stroke limiter 128, 158 does abut against the barrel abutment surface of the syringe barrel 106 before the stopper has reached a maximum end position where the volume of the reservoir containing the medical product is minimal.

Before performing the injection, the end-user of the syringe assembly 100 determines what fraction of the maximum dose of medical product has be to be injected to the patient. The end user then adjusts the position of the plunger stroke limiter 128, 158 with respect to the stopper mount portion 123 along the longitudinal axis A, to the position corresponding to that faction. Then, the end-user can perform the injection without having to monitor the degree of displacement of the piston assembly 104. The end-user can be confident that the desired dose of medical product will have been delivered to the patient when the limited stroke of the plunger assembly 104 is reached, i.e. when the plunger stroke limiter 128, 158 abuts against the syringe body abutment surface 130 of the syringe body, thereby stopping any further displacement of the piston assembly 104.

Thus, the plunger assembly as recited above allows for an easy and reliable procedure of injection of the desired fraction of the maximum dose of medical product, letting the end-user dedicate his/her attention to other aspects of the injection procedure.

It is to be understood that the present invention is not limited to the embodiments described above and illustrated in the drawings; rather, the skilled person will recognize that changes and modifications may be made within the scope of the appended claims.

## Claims

1. Plunger assembly (104) for a syringe assembly (100) comprising a syringe body (102), wherein the plunger assembly (104) comprises:
- a plunger rod (102) extending along a longitudinal axis (A) and having a proximal end (126) and a distal end (123);
- at the distal end of the plunger rod, a stopper mount portion (123) for receiving a stopper (124);
**characterized in that** the plunger assembly (104) comprises a plunger stroke limiter (128, 158), **in that** the position of the plunger stroke limiter (128, 158) with respect to the stopper mount portion (123) is user-adjustable along the longitudinal axis, and **in that** the plunger stroke limiter (123) is configured to abut against a syringe body abutment surface (130) of the syringe body (102) for at least one position of the plunger stroke limiter (128, 158) with respect to the stopper mount portion (123).

2. Plunger assembly according to claim 1, **characterized in that** the plunger stroke limiter (128) is mounted on the plunger rod (122) in one of at least two distinct positions with respect to the stopper mount portion (123) along the longitudinal axis (A), and **in that** the position of plunger stroke limiter (128) is user-adjustable to the other of the at least two distinct positions.

3. Plunger assembly according to any preceding claim, **characterized in that** the plunger stroke limiter comprises a ring (132) which is co-axially mounted on the plunger rod (122).

4. Plunger assembly according to any preceding claim, **characterized in that** the plunger stroke limiter (128) is mounted on the plunger rod (122) via a helical thread (134).

5. Plunger assembly according to claim 1, **characterized in that** the plunger rod (122) is formed of two parts comprising a proximal part (156) and a distal part (154) which can be connected one to the other according to at least two distinct positions with respect one to the other along the longitudinal axis (A), **in that** the stopper mount portion (123) is formed on the proximal part (154), and **in that** the plunger stroke limiter (158) is formed on the distal part (156).

6. Plunger assembly according to claim 5, **characterized in that** the distal part (154) and the proximal part (156) of the plunger rod are telescopically connected.

7. Plunger assembly according to claim 6, **characterized in that** the distal part (154) and the proximal part (156) of the plunger rod (122) are telescopically connected via a helical thread (134, 160).

8. Plunger assembly according to any of claims 5 to 7, **characterized in that** the proximal part (156) of the plunger rod has a proximal end having comprises a radial plunger flange (126), and **in that** the radial plunger flange (126) forms the plunger stroke limiter (158).

9. Plunger assembly according to any preceding claim, **characterized in that** the position of the plunger stroke limiter (128, 158) with respect to the stopper mount portion (123) along the longitudinal axis (A) is secured by a ratchet mechanism (140, 141, 138).

10. Plunger assembly according to claim 9, **characterized in that** the ratchet mechanism is an asymmetric ratchet requiring a greater force to adjust a decrease of longitudinal distance between the plunger stroke limiter (128, 158) and the stopper mount portion (123) than a force needed to adjust an increase of longitudinal distance between the plunger stroke limiter (128, 158) and the stopper mount portion (123), or a one-way irreversible ratchet allowing only an increase of the distance between the plunger stroke limiter (128, 158)and the stopper mount portion (123).

11. Plunger assembly according to any preceding claim, **characterized in that** the plunger stroke limiter (128) comprises at least one lock (162) which is adapted for locking the plunger stroke limiter (128) on the syringe body (102).

12. Plunger assembly according to any preceding claim, **characterized in that** it further comprises a stopper (124) mounted on the stopper mount portion (123) of the plunger rod (122).

13. Plunger assembly according to claim 12, **characterized in that** the stopper (124) comprises at least one circular sealing rib (125) having a sealing radius along a radial direction perpendicular to the longitudinal axis (A) **in that** the plunger stroke limiter (128, 158) has a maximal radial extent with respect to the longitudinal axis (A) which is greater than the sealing radius.

14. Syringe assembly (100) having a syringe body (102) and plunger assembly (104) according to any preceding claim.

15. Syringe assembly according to claim 14, **characterized in that** the syringe body comprises a syringe barrel which has a tubular shape along the longitudinal axis and an open proximal end defining a proximal facing end surface having an internal diameter around the longitudinal axis, wherein the maximum radial extent of the plunger stroke limiter is greater that than the internal diameter of the proximal facing end surface of the syringe barrel.
